# EUROPÄISCHE PATENTSCHRIFT

(11) **EP 3 823 564 B1**
(45) Veröffentlichungstag und Bekanntmachung des Hinweises auf die Patenterteilung: **13.09.2023**
(21) Anmeldenummer: 19744627.1
(22) Anmeldetag: 03.07.2019
(51) Int. Cl.: A61F 2/60, A61F 2/64, A61F 2/66, A61F 2/68

(54) **ORTHOPÄDIETECHNISCHE VORRICHTUNG MIT EINEM FUSSTEIL, EINEM UNTERSCHENKELTEIL UND EINEM OBERSCHENKELTEIL**
ORTHOPEDIC DEVICE, HAVING A FOOT PART, A LOWER-LEG PART AND A THIGH PART
DISPOSITIF ORTHOPÉDIQUE COMPORTANT UNE PARTIE PIED, UNE PARTIE JAMBE ET UNE PARTIE CUISSE

(30) Priorität: 18.07.2018 DE 102018117377
(43) Veröffentlichungstag der Anmeldung: 26.05.2021
(73) Patentinhaber: Ottobock SE & Co. KGaA, 37115 Duderstadt (DE)
(72) Erfinder: BELLMANN, Malte, 34346 Hann.Münden (DE); BOITEN, Herman, 6715 LE Ede (NL); WEBER, Paul, 65187 Wiesbaden (DE)
(74) Vertreter: Gramm, Lins & Partner Patent- und Rechtsanwälte PartGmbB
(86) Internationale Anmeldenummer: PCT/EP2019/067887
(87) Internationale Veröffentlichungsnummer: WO 2020/016014

(56) Entgegenhaltungen:
- EP-A1- 3 257 478
- EP-A2- 0 549 855
- EP-A2- 1 447 062
- WO-A1-2012/177125

## Beschreibung

Die Erfindung betrifft eine orthopädietechnische Vorrichtung mit einem Fußteil, einem Unterschenkelteil und einem Oberschenkelteil, wobei das Fußteil mit dem Unterschenkelteil über ein Knöchelgelenk verschwenkbar um eine erste Schwenkachse verbunden und das Unterschenkelteil mit dem Oberschenkelteil über ein Kniegelenk schwenkbar um eine zweite Schwenkachse verbunden ist, wie in den Ansprüchen definiert. Das Fußteil ist zudem mit dem Oberschenkteil über eine Kraftübertragungseinrichtung verbunden. Eine solche orthopädietechnische Vorrichtung ist insbesondere als eine Prothese oder eine Orthese ausgestaltet. Bei einer Ausgestaltung als Prothese ist das Oberschenkelteil bevorzugt als ein Oberschenkelschaft zur Aufnahme eines Stumpfes oder als ein Prothesenkniegelenkoberteil ausgebildet, das mit einem solchen Oberschenkelschaft verbindbar ist. Bei einer Ausgestaltung einer orthopädietechnischen Vorrichtung als Orthese ist an dem Oberschenkelteil zumindest eine Befestigungseinrichtung angeordnet, über die das Oberschenkelteil, beispielsweise eine Oberschenkelschiene oder eine Oberschenkelschale, an einem Oberschenkel eines Patienten anlegbar ist.

Orthesen haben die Aufgabe, die Bewegung einer vorhandenen Gliedmaße zu führen oder zu unterstützen beziehungsweise eine Gliedmaße abzustützen und zu unterstützen. Orthesen für die untere Extremität sind in unterschiedlichen Ausführungsformen bekannt. Sogenannte KAFO (knee ankle foot orthosis) stützen sowohl den Fuß als auch das Knöchelgelenk und das Kniegelenk ab. Der Fuß wird in der Regel auf einer Fußplatte aufgesetzt, eine oder mehrere Unterschenkelschienen erstrecken sich parallel zu dem Unterschenkel und ein Orthesenkniegelenk ist ungefähr im Bereich der natürlichen Knieachse vorgesehen. An einer oder an mehreren Oberschenkelschienen sind Befestigungseinrichtungen zum Festlegen der Orthese an dem Oberschenkel angebracht. Ebenfalls können Befestigungseinrichtungen an dem Unterschenkelteil oder der Fußplatte vorgesehen sein, um die Orthese an dem jeweils zu versorgenden Bein festlegen zu können.

Prothesen mit einem Prothesenkniegelenk weisen einen Prothesenfuß als Fußteil auf, der über ein Unterschenkelrohr als Unterschenkelteil mit dem Prothesenkniegelenk verbunden ist. Proximal zu der Prothesenkniegelenksachse ist eine Befestigungseinrichtung für die Prothese vorgesehen, um die Prothese an dem Oberschenkelstumpf festzulegen. Als Prothesenkniegelenk können unterschiedliche Bauformen eingesetzt werden, beispielsweise monoaxiale Prothesenkniegelenke, polyzentrische Kniegelenke mit Dämpfungseinrichtungen oder computergesteuerte und angetriebene aktive Prothesenkniegelenke.

Aus der DE 10 2012 023 023 A1 sowie aus der EP3257478 A1 ist eine orthopädietechnische Vorrichtung zur orthetischen oder prothetischen Versorgung eines Patienten mit einem Kniegelenk, das ein proximales Oberteil und ein schwenkbar um eine Knieachse daran angeordnetes distales Unterteil aufweist, bekannt. Weiterhin ist ein Knöchelgelenk mit einer Knöchelgelenksachse und einem distal an dem Knöchelgelenk angeordneten, um die Knöchelgelenkachse verschwenkbaren Fußteil vorgesehen. Zwischen dem Knöchelgelenk und dem Kniegelenk ist ein Unterschenkelteil angeordnet. Um bei einem möglichst geringen konstruktiven Aufwand eine Kopplung zwischen dem Kniegelenk und dem Knöchelgelenk herbeizuführen und die Nutzung der Bewegungsenergie des Knies für eine Knöchelbewegung zu ermöglichen, damit eine Annäherung an das natürliche Gangbild bewirkt wird, ist das Oberteil des Kniegelenkes mit dem Fußteil über eine Kraftübertragungseinrichtung gekoppelt, bei der bei einer Knieflexion eine Plantarflexion des Fußteils bewirkt wird. Dadurch wird am Ende der Standphase, bei der eine Knieflexion eintritt, eine Plantarflexion vorgenommen, um die Beinlänge bei der Kniebeugung zu verlängern. Dadurch wird die Dauer des Bodenkontaktes des Fußteils verlängert und die Vertikalbewegung des Körperschwerpunktes minimiert.

Aus der US 2008/0269913 A1 ist ein Kunstbein mit einem Prothesenkniegelenk und einem Prothesenfuß bekannt. An dem Prothesenkniegelenk ist eine Verbindungsstange frontal zu der Kniegelenksachse befestigt, sodass bei einer Knieflexion die Verbindungsstange in einer Führung im Unterschenkel verschoben wird. Über ein Zugelement wird die Bewegung zum Prothesenfuß geleitet, sodass die Fußspitze bei einer Knieflexion angehoben wird.

Die EP 0 041 052 B1 betrifft eine Prothese für eine untere Gliedmaße, bei der ein Oberschenkelschaft und ein Unterschenkel über ein Verzahnungsgelenk miteinander gekoppelt sind. Eine federbelastete Kolbenstange hebt bei einer Knieflexion die Zehen an.

Die DE 47 53 03 B1 betrifft ein künstliches Bein, bei dem ein Unterschenkelteil mit einem Oberschenkelteil durch zwei Gelenkstäbe miteinander verbunden sind, um bei einem Anwinkeln des Prothesenkniegelenkes eine Dorsalflexion zu bewirken.

Die Kopplung einer Dorsalflexion bei einer Knieflexion wird vorgenommen, um das Durchschwingen eines künstlichen Beines zu erleichtern. Wird die Fußspitze im Rahmen einer Dorsalflexion während der Schwungphase nicht angehoben, besteht die Gefahr, dass die Fußspitze an dem Boden schleift und hängen bleibt. Vielfach wird dies durch ein unnatürliches Gangbild ausgeglichen, bei dem eine Zirkumduktion stattfindet.

Die EP 0 549 855 A2 betrifft eine Oberschenkelknieprothese mit einem Oberschenkel- und einem Unterschenkelteil, einem diese verbindenden Kniegelenk, einem linearen hydraulischen Dämpfer zur Dämpfung der Verschwenkbewegungen des Kniegelenkes und mit einer den Dämpfer beaufschlagenden Verstelleinrichtung zur Veränderung der Kniegelenkdämpfung. Eine elektronische Messeinrichtung misst den jeweiligen Kniewinkel und die Unterschenkelteil-Belastung und übermittelt die Messwerte an einen programmierten Rechner zum Vergleich mit gespeicherten Schwellwerten, die kennzeichnend sind für vorgegebene, zur Verstellung von zumindest einer der beiden Beugungs- und Streckdämpfungen ausgewählten Übergangspunkte. Die Verstelleinrichtung wird zur Veränderung der Dämpfung aktiviert, wenn die Sensorwerte mit den gespeicherten Werten übereinstimmen.

Die EP 1 447 062 A2 betrifft eine Beinprothese mit einem Oberschenkelschaft, einem Prothesenkniegelenk, einem Unterschenkelteil sowie einem gelenkig an dem Unterschenkelteil befestigten Prothesenfuß. Das Prothesenkniegelenk ist so ausgebildet, dass es bei einer Flexionsbewegung um eine Schwenkachse eine kombinierte Roll-Gleit-Bewegung ausführt, sodass sich der Abstand eines dorsal gesehen vor der Schwenkachse gelegenen Punktes zum distalen Ende des Prothesenunterschenkels hin verkleinert, während sich der Abstand eines ventral gesehen vor der Schwenkachse liegenden Punktes vergrößert. Weiterhin ist ein Kraftübertragungselement zwischen einem der beiden Punkte und dem Prothesenfuß angeordnet, das den Prothesenfuß beim Beugen des Prothesenkniegelenkes in zunehmendem Maße aus einer Spitzfußlage in eine Hackenfußlage überführt.

Nutzer einer orthopädietechnischen Vorrichtung für eine untere Extremität müssen neben Herausforderungen beim Gehen auch mit Herausforderungen beim Sitzen, Hinsetzen und Aufstehen umgehen, da bei Nutzern orthopädietechnischer Vorrichtungen der unteren Extremität Muskeln in ihrer Funktion eingeschränkt oder nicht vorhanden sind.

Aufgabe der vorliegenden Erfindung ist es, eine orthopädietechnische Vorrichtung bereitzustellen, mit der das Hinsetzen und Aufstehen erleichtert wird.

Erfindungsgemäß wird diese Aufgabe durch eine Vorrichtung mit den Merkmalen des Hauptanspruchs gelöst. Vorteilhafte Ausgestaltungen und Weiterbildungen der Erfindung sind in den Unteransprüchen, der Beschreibung sowie den Figuren offenbart.

Die erfindungsgemäße orthopädietechnische Vorrichtung mit einem Fußteil, einem Unterschenkelteil und einem Oberschenkelteil, wobei das Fußteil mit dem Unterschenkelteil über ein Knöchelgelenk schwenkbar um eine erste Schwenkachse verbunden ist, das Unterschenkelteil mit dem Oberschenkelteil über ein Kniegelenk schwenkbar um eine zweite Schwenkachse verbunden ist und das Fußteil mit dem Oberschenkelteil über eine Kraftübertragungseinrichtung verbunden ist, sieht vor, dass die Kraftübertragungseinrichtung bei einer Knieflexion über einen ersten Knieflexionswinkelbereich eine Dorsalflexion des Fußteils und nach Überschreiten eines Knieflexionsgrenzwinkels bei weiterer Knieflexion eine Plantarflexion des Fußteils bewirkt. Setzt sich ein Nutzer einer orthopädietechnischen Vorrichtung hin, bewegt sich das Oberschenkelteil um die Kniegelenkachse. Der Körperschwerpunkt wird ebenfalls im Rahmen einer Kreisbewegung um die Kniegelenkachse verschwenkt, was dazu führt, dass der Körperschwerpunkt sehr schnell aus dem Bereich der Unterstützungsfläche des Fußteils herausbewegt wird. Der Körperschwerpunkt liegt dann hinter der Unterstützungsfläche, was dazu führt, dass der gesamte Körper nach hinten kippt. Dies muss durch einen Nutzer der orthopädietechnischen Vorrichtung durch ein gesundes Bein oder durch zur Hilfenahme von Unterstützungseinrichtungen oder den Armen ausgeglichen werden. Durch eine zwangsweise Dorsalflexion des Fußteils findet eine Vorwärtsrotation des Unterschenkelteils um die Knöchelgelenksachse statt, sodass die Kniegelenksachse nach vorne bewegt wird. Diese Bewegung führt dazu, dass der Körperschwerpunkt über eine gewissen Knieflexionsbereich nach vorne unter die Unterstützungsfläche des Fußteils verlagert wird, sodass eine Neigung, nach hinten zu kippen, unterdrückt oder zumindest vermindert wird. Nach Überschreiten eines Knieflexionsgrenzwinkels, der festlegbar ist, wird bei einer weiteren Knieflexion eine Plantarflexion des Fußteils bewirkt. Diese Plantarflexion führt bei dem Hinsetzvorgang weiterhin aufgesetzten Fußteil dazu, dass die Schwenkbewegung des Unterschenkelteils relativ zu dem Fußteil in die entgegengesetzte Richtung, also nach hinten verschwenkt wird, wodurch der Körperschwerpunkt aufgrund der Schwenkbewegung der Kniegelenkachse weiter nach hinten verlagert wird. Der Knieflexionsgrenzwinkel liegt in einem Bereich zwischen 50° und 80°, insbesondere zwischen einem Bereich von 60° und 80°, insbesondere bei 75°.

Darüber hinaus hilft die Plantarflexion dabei, dass sich der Patient so hinsetzen kann, dass er auf der gewünschten Position in Kontakt mit der Sitzfläche kommt und nicht auf dem vorderen Rand der Sitzfläche landet. Während der Knieflexion beim Hinsetzen werden nacheinander bei einer zunehmenden Knieflexion, also einer Verringerung des eingeschlossenen Winkels zwischen der in Gehrichtung hinteren Oberschenkelseite und Unterschenkelseite, zwei einander entgegengesetzte Bewegungen mit einer Bewegungsumkehr bei Erreichen eines Knieflexionsgrenzwinkels durch das Unterschenkelteil um die Knöchelgelenksachse durchgeführt. Zunächst findet eine Verschwenkung des Unterschenkelteils um die Knöchelgelenksachse in Gehrichtung, also nach vorne statt, wodurch die Kniegelenksachse in Gehrichtung nach vorne verlagert wird, nach Erreichen des Knieflexionsgrenzwinkels wird die Verschwenkbewegung um die Knöchelgelenkachse umgekehrt und die Kniegelenksachse und damit auch der Körperschwerpunkt entgegen der Gehrichtung, also nach hinten verlagert.

Die Kraftübertragungseinrichtung kann als hydraulisches System oder als zugkraft- und druckkraftübertragende mechanische Koppeleinrichtung ausgebildet sein. Bei einer Ausgestaltung als zugkraft- und druckkraftübertragende mechanische Koppeleinrichtung ergibt sich der Vorteil eines geringen konstruktiven Aufwandes und einer leichten Nachrüstbarkeit. Übersetzungsverhältnisse lassen sich durch Längenveränderungen leicht anpassen. Über ein hydraulisches System mit Zylindern und Kolben, Leitungen und Umschaltventilen kann eine platzsparende, leichte Kraftübertragung von dem Kniegelenk zu dem Fußteil erfolgen. Die Bewegungsumkehr kann über ein Umschaltventil durchgeführt werden, das mechanisch und/oder elektrisch angesteuert werden kann.

Bei einer Ausgestaltung der Kraftübertragungseinrichtung als mechanische Koppeleinrichtung kann eine erste Lagerstelle beabstandet zu der Schwenkachse an dem Fußteil und an einer zweiten Lagerstelle beabstandet zu der zweiten Schwenkachse an dem Oberschenkelteil gelagert sein, wobei die erste Lagerstelle bei Erreichen des Knieflexionsgrenzwinkels eine maximale distale oder proximale Position einnimmt. Durch die Festlegung der Position der maximalen distalen oder proximalen Position der ersten Lagerstelle bei Erreichen des Knieflexionsgrenzwinkels wird die Position der Bewegungsumkehr der Koppeleinrichtung definiert. Je nach Anordnung der mechanischen Koppeleinrichtung an dem Fußteil, also in Gehrichtung vor oder hinter der Knöchelgelenkachse, werden unterschiedliche Bewegungen durch eine Verschwenkung um die Kniegelenkachse hervorgerufen. Die erste Lagerstelle führt eine Kreisbewegung um die Kniegelenkachse aus und erreicht bei dem festgelegten Knieflexionsgrenzwinkel den maximalen oder distalen Scheitelpunkt der Trajektorie. Bei einer fortgesetzten Knieflexion führt dies zu einer Bewegungsumkehr entweder in Richtung auf die Knöchelgelenkachse hin oder von dieser weg, sodass nach Überschreiten des Knieflexionsgrenzwinkels eine Plantarflexion ausgeführt wird. Liegt beispielsweise eine Ausgangsposition, bei der der Kniewinkel maximal ist, die zweite Lagerstelle in Gehrichtung vor der Kniegelenksachse, befindet sich die erste Lagerstelle an dem Fußteil ebenfalls in Gehrichtung vor der Knöchelgelenksachse, sodass bei Erreichen des Knieflexionsgrenzwinkels die zweite Lagerstelle maximal proximal positioniert ist. Dementsprechend ist die zweite Lagerstelle maximal von der Knöchelgelenksachse entfernt und der Knöchelgelenkswinkel oder Plantarflexionswinkel ist minimal. Anschließend wird bei einer weiteren Knieflexion der Flexionswinkel weiter verringert und aufgrund der Kreisbahnbewegung oder angenäherten Kreisbahnbewegung der zweiten Lagerstelle die Koppeleinrichtung wieder in Richtung auf die Knöchelgelenkachse verlagert, was zu einer Plantarflexion führt. Dementsprechend befindet sich bei Erreichen des maximalen Knieflexionswinkels auch die erste Lagerstelle maximal proximal, sodass sich eine maximale Dorsalflexion einstellt, die bei einer weiteren Flexion umgekehrt in eine Plantarflexion umgewandelt wird. Befindet sich die zweite Lagerstelle in der gestreckten Position des Kniegelenkes hinter der Kniegelenksachse, ist die erste Lagerstelle ebenfalls hinter der Knöchelgelenksachse angeordnet, sodass beide Lagerstellen hinter der Verbindungslinie zwischen der Kniegelenksachse und der Knöchelgelenksachse befindlich sind. Wird durch die Knieflexionsbewegung die zweite Lagerstelle in eine maximal distale Position gebracht, befindet sich auch die erste Lagerstelle in einer maximalen distalen Position und das Fußteil in einer maximal dorsal flektierten Position. Eine maximale distale Position nimmt die zweite Lagerstelle ein, wenn sie auf der Verbindungslinie zwischen der Kniegelenksachse und der Knöchelgelenksachse liegt, eine maximal proximale Position nimmt die zweite Lagerstelle ein, wenn sie auf der Verbindungslinie zwischen der Knöchelgelenkachse und der Kniegelenkachse proximal der Kniegelenksachse befindlich ist.

Die Position zumindest einer der Lagerstellen ist in einer Weiterbildung der Erfindung einstellbar, um das Maß der Dorsalflexion oder Plantarflexion einzustellen, also um die Hebelverhältnisse einstellen zu können. Darüber hinaus kann die Position der Lagerstellen hinsichtlich ihrer Winkelstellung eingestellt werden, um beispielsweise den Knieflexionsgrenzwinkel einzustellen. Ist beispielsweise die zweite Lagerstelle um die Kniegelenksachse verdrehbar und in einer bestimmten, wählbaren Position festlegbar, kann darüber der Knieflexionsgrenzwinkel eingestellt werden, also derjenige Winkel, ab dem eine Vorverlagerung der Kniegelenksachse in eine Rückverlagerung umgekehrt wird. Eine Einstellung kann auch durch eine Veränderung der Länge der Kraftübertragungseinrichtung erfolgen.

Die Lagerstellen können auf einer Kreisbahn geführt sein, alternativ dazu ist es möglich, eine Kulissenführung der Lagerstellen vorzusehen, sodass sich eine nahezu beliebige Zuordnung eines Kniewinkelverlaufes zu einem Knöchelwinkelverlauf einstellen lässt.

Die Lagerstellen können abnehmbar an dem Fußteil und/oder dem Oberschenkelteil befestigt sein, um bereits vorhandenen Prothesenkniegelenke oder Orthesenkniegelenke mit daran angeschlossenen Fußteilen nachrüsten zu können. Dies ist bei einer mechanischen Ausgestaltung der Kraftübertragungseinrichtung leicht möglich. Sofern an einem Fußteil oder einem Oberschenkel bereits eine Lagerstelle für ein Koppelelement angeordnet oder ausgebildet ist, kann die noch fehlende Lagerstelle einzeln nachgerüstet werden, um aus einer bereits vorhandenen orthopädietechnischen Vorrichtung ohne eine Hinsetz- und Aufstehhilfe eine erfindungsgemäße Vorrichtung herstellen zu können.

Die Länge der Koppeleinrichtung zwischen den Lagerstellen ist einstellbar, um eine individuelle Anpassung an den jeweiligen Patienten vornehmen zu können.

Eine Weiterbildung der Erfindung sieht vor, dass zwischen dem Unterschenkelteil und dem Oberschenkelteil ein Energiespeicher und/oder eine Dämpfereinrichtung angeordnet sind. Dadurch ist es möglich, einerseits die Bewegung beim Hinsetzen zu dämpfen, um ein zu schnelles Absacken des Körpers zu verhindern. Andererseits ist bei einer Vorrichtung mit Energiespeicher, beispielsweise einer Feder, eine Aufstehunterstützung gegeben, mit der einem Patienten das Aufstehen erleichtert wird. Wird der Energiespeicher beim Hinsetzen aufgeladen, kann durch eine Bewegungsumkehr dieser freigegeben werden, um eine Aufstehunterstützung zu liefern.

Um die Kniebeugung beim Hinsetzen zu kontrollieren, ist in einer Ausführungsform der Erfindung in der orthopädietechnischen Vorrichtung ein Dämpfungselement angeordnet. Somit entsteht eine Unterstützung zur kontrollierten Absenkung des Körperschwerpunktes bei zunehmender Knieflexion. In einer Weiterbildung der Erfindung kann das Dämpfungselement als progressiv wirkendes Dämpfungselement ausgebildet sein, das bei einem zunehmenden Kniebeugewinkel, also bei einer zunehmenden Knieflexion, über eine progressive Zunahme der generierten Kraft verfügt. Mit zunehmender Knieflexion erhöht sich somit die Dämpfungskraft, die durch das Dämpfungselement aufgebracht wird. Das Maß der Progression der Dämpfungskraft kann entweder über eine mechanische Auslegung der Dämpfereinrichtung erfolgen, bei der die Konturen des Kolbens und/oder des Zylinders und/oder Bypässe bei einem zunehmenden Flexionswinkel den Strömungswiderstand erhöhen oder über eine mechatronische Ansteuerung eines Ventils, z.B. ein Stellventil oder ein verstellbares Drosselventil, zur Veränderung des Hydraulikwiderstandes erfolgen. Die Dämpfereinrichtung weist dabei einen vergleichsweise niedrigen Anfangswiderstand bei einem extendierten, also maximal gestreckten Kniegelenk auf und steigt auf einen sehr hohen Widerstand in einem Kniewinkelbeugebereich zwischen 70 ° und 90° an. Bevorzugt ist die Progression stufenlos. Die Höhe des Dämpfungsniveaus ist einstellbar, damit die Vorrichtung an unterschiedliche Patientengewichte angepasst werden kann. Die Anpassung und Verstellung des Dämpfungsniveaus kann über ein manuell einstellbares Ventil oder eine Drossel oder über eine Programmierung eines mechatronisch angesteuerten Ventils erfolgen.

Das Dämpferelement kann auch zur vollständigen Sperrung des Kniegelenkes gegen ein ungewolltes Einbeugen des Kniegelenkes beim Gehen oder Stehen genutzt werden, um eine ungewollte oder unkontrollierte Einbeugung des Kniegelenkes zu verhindern. Bei einem hydraulischen Dämpferelement wird ein Überströmen aus einer Extensionskammer in eine Flexionskammer oder umgekehrt gesperrt, so dass eine Knieflexion vollständig verhindert wird. Das Abschalten der Sperrung der Knieflexion und die dann damit einhergehende Verringerung des Beugewiderstandes oder eine verringerte Beugedämpfung für das Hinsetzen kann entweder durch einen manuellen Schalter oder eine mechatronische Erkennung des Hinsetzvorganges erfolgen, beispielsweise über eine bewegungsbasierte Steuerung oder über eine Steuerung, die über Belastungssensoren erkennt, wann eine Hinsetzbewegung erfolgt.

Ebenso kann das Aufstehen durch einen hydraulisch realisierten Sperrvorgang bei einer Bewegungsumkehr, ähnlich einem Ratschenmechanismus einer mechanischen Lösung, unterstützt werden, indem eine erneute Flexion und Kniebeugung nach einer Unterbrechung der Extensionsbewegung während des Aufstehens gesperrt wird. Somit ist es einem Nutzer der orthopädietechnischen Vorrichtung möglich, Last auf eine eingebeugte Prothese oder Orthese zu verlagern und so den Aufstehvorgang in mehreren Schritten durchzuführen. Ab einem Flexionswinkel von 20° bis 30°, also einem verbleibenden Extensionswinkel von 20° bis 30° bis zur maximal extendierten Streckposition kann dieser hydraulische Ratschen-Mechanismus wieder deaktiviert werden. Der Mechanismus oder die Schaltung kann entweder mechanisch über die Gestaltung der Hydraulik oder mechatronisch über eine bewegungsabhängige Schaltung eines Ventils auf Basis von Sensordaten verwirklicht werden.

Die Hydraulik kann weiterhin so gestaltet sein, dass eine Erhöhung der Extensionsdämpfung vor Erreichen des mechanischen Streckanschlages, also vor Erreichen der mechanisch vorgegebenen maximalen Extension vorhanden ist, um einen harten Anschlag an den Extensionsanschlag beim Aufstehen zu dämpfen, um den Komfort für den Patienten zu erhöhen. Eine Streckanschlagdämpfung kann entweder mechanisch über eine Kolbengeometrie oder ein Elastomerelement oder mechatronisch über eine winkelabhängige Ansteuerung eines Ventils umgesetzt werden.

Nach Erreichen des für das Sitzen üblicherweise benötigten Flexionswinkels oder Kniebeugewinkels kann die Flexionsdämpfung reduziert werden, gegebenenfalls aufgehoben werden, um dem Unterschenkel oder dem Unterteil nach dem Anheben der Prothese oder Orthese ein freies Pendeln zu ermöglichen. Somit kann der Patient oder Nutzer der orthopädietechnischen Vorrichtung beim Sitzen den Unterschenkel einfach in die gewünschte Position bringen.

Der jeweiligen Gelenkeinrichtung kann ein Energiespeicher zugeordnet werden, um beispielsweise Energie beim Hinsetzen zu speichern und diese zur Unterstützung der Aufstehbewegung wieder abzugeben. Zudem kann dem Energiespeicher eine Sperre zugeordnet sein, die verhindert, dass die gespeicherte Energie z.B. im Sitzen zum falschen Zeitpunkt freigegeben wird. Diese Sperre kann entweder manuell oder über einen sensorgesteuerten Aktuator geöffnet werden, um das Aufstehen zu einem gewünschten Zeitpunkt zu unterstützen. Dadurch ist es möglich, einmal gespeicherte Energie beim Aufstehen oder für das Aufstehen freizuschalten, um eine Aufstehbewegung zu unterstützen.

Dem Knöchelgelenk und/oder dem Kniegelenk kann eine betätigbare Sperreinrichtung zugeordnet sein, die ein Einbeugen des Gelenks verhindert und ein sicheres Gehen mit einem steifen Bein ermöglicht. Zum Hinsetzen wird diese Sperre manuell oder über Sensoren und eine Aktuatorik entriegelt. Eine Verriegelungseinrichtung, die eine Flexion des Kniegelenkes sperrt, erlaubt das Gehen mit einem Prothesenbein, ohne dass das Risiko eines Einbeugens oder Einknickens besteht.

Nachfolgend werden Ausführungsbeispiele der Erfindung anhand der beigefügten Figuren näher erläutert. Es zeigen:
- Figur 1 -: eine Seitenansicht einer orthopädietechnischen Vorrichtung in Gestalt einer Prothese in gestreckter Stellung;
- Figur 2 -: eine Darstellung in gebeugter Stellung bei Knieflexionsgrenzwinkel;
- Figur 3 -: eine Seitenansicht in Sitzposition;
- Figur 4 -: eine Darstellung gemäß Figur 2 mit Winkelangaben;
- Figur 5 -: ein Diagramm des Knöchelwinkels über den Kniewinkel;
- Figur 6 -: zwei Verlaufsdarstellungen eines Hinsetzvorganges;
- Figur 7 -: eine Variante in Teilschnittdarstellung mit einem Dämpfer und Sperreinrichtung;
- Figur 8 -: eine Variante der Vorrichtung mit Energiespeicher;
- Figur 9 -: eine weitere Variante der Erfindung; sowie
- Figur 10 -: eine Variante der Figur 9 in gebeugter Stellung bei Knieflexionsgrenzwinkel;
- Figur 11 -: eine Detailansicht der Figur 7;
- Figur 12 -: eine Variante der Erfindung gemäß Figur 11 in geöffneter Stellung, sowie
- Figur 13 -: eine Variante der Erfindung mit einer Verriegelungseinrichtung an einem Energiespeicher;
- Figur 14 -: eine schematische Darstellung eines hydraulischen Systems zur Kraftübertragung;
- Figur 15 -: eine Variante der Figur 14; sowie
- Figur 16 -: eine schematische Darstellung einer Variante eines hydraulischen Systems.

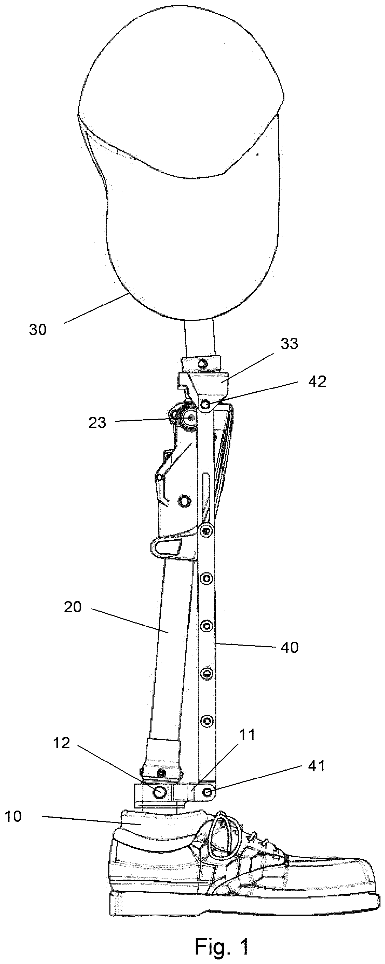

In der Figur 1 ist in einer Seitenansicht eine orthopädietechnische Vorrichtung in Gestalt einer Prothese einer unteren Extremität mit einem Fußteil 10, einem Unterschenkelteil 20 und einem Oberschenkelteil 30 gezeigt. Das Oberschenkelteil 30 ist als Prothesenschaft mit einem angesetzten Rohrstück zur Verbindung mit einem Oberteil eines Prothesenkniegelenkes ausgebildet. Das Unterschenkelteil 20 weist ein Unterschenkelrohr und Unterteil eines Prothesenkniegelenkes auf und ist mit dem Oberschenkelteil 30 um eine zweite Schwenkachse 23 schwenkbar verbunden. Das Fußteil 10 mit einem Prothesenfuß, der in einem Schuh eingeführt ist, ist schwenkbar um eine erste Schwenkachse 12 mit dem Unterschenkelteil 20 verbunden. Die orthopädietechnische Vorrichtung befindet sich in der gestreckten, maximal extendierten Stellung. An dem Fußteil 10 ist ein erster Ausleger 11 starr befestigt. Innerhalb des Auslegers 11 verläuft die erste Schwenkachse 12, sodass das Unterschenkelteil 20 relativ zu dem Fußteil 10 um die erste Schwenkachse 12 verschwenken kann, wenn das Fußteil 10 auf dem Boden flach aufgesetzt ist. Der erste Ausleger 11 kann lösbar an dem Fußteil 10 befestigbar und daran festlegbar ausgebildet sein. Dadurch ist es möglich, den Ausleger 11 auch nachträglich an eine fertige orthopädietechnische Vorrichtung, beispielsweise eine Prothese oder eine Orthese, anzubringen. In dem dargestellten Ausführungsbeispiel ist an dem ersten Ausleger 11 in Frontalrichtung beabstandet zu der ersten Schwenkachse 12 eine erste Lagerstelle 41 ausgebildet, die in normaler Gehrichtung vor der Schwenkachse 12 liegt. An der ersten Lagerstelle 41 ist eine Kraftübertragungseinrichtung 40 in Gestalt eines mechanischen Koppelelementes angeordnet, die sich in Proximalrichtung erstreckt. Das Koppelelement 40 ist in der Länge einstellbar. Dazu ist das Koppelelement 40 zweiteilig ausgebildet und weist in dem proximalen Teil eine Langlochführung auf, entlang der das distale Teil verschoben werden kann. Über Schrauben kann dann in der jeweils gewünschten Position das erste Teil an dem zweiten Teil fixiert werden. Das proximale Ende des Koppelelementes 40 ist an einer zweiten Lagerstelle 42 befestigt, die an einem zweiten Ausleger 33 ausgebildet ist, der an dem Oberteil 30 befestigt ist. Der zweite Ausleger 33 kann ebenfalls lösbar an dem Oberschenkelteil 30 oder dem Oberteil des Prothesenkniegelenkes befestigt werden, um eine Nachrüstung zu ermöglichen. Die zweite Lagerstelle 42 befindet sich in der dargestellten extendierten Stellung der orthopädietechnischen Vorrichtung in Gehrichtung vor der zweiten Schwenkachse 23, sodass beide Lagerstellen 41, 42 frontal vor der Verbindungslinie zwischen den beiden Schwenkachsen 12, 23 an dem Knöchelgelenk und dem Kniegelenk liegen. Die zweite Lagerstelle 42 befindet sich näher an der zweiten Schwenkachse 23 als die erste Lagerstelle 41 zu der ersten Schwenkachse 12. Über die Längenverhältnis, also über die Abstände der Lagerstellen 41, 42 zu den jeweiligen Schwenkachsen 12, 23 kann eine Übersetzung stattfinden. Je kürzer der Abstand der zweiten Lagerstelle 42 zu der zweiten Schwenkachse 23 ist, desto geringer ist der zurückgelegte Weg auf der Kreisbahn und desto geringer ist der Verschwenkwinkel des Unterschenkelteils 12 relativ zu dem statischen Fußteil 10. Die erste Lagerstelle 41 liegt auf einer gemeinsamen Ebene mit der ersten Schwenkachse 12, die im Wesentlichen parallel zu einem ebenen Fußboden verläuft, die zweite Lagerstelle 42 ist posterior zu der zweiten Schwenkachse 23 positioniert, also um einen Winkel gegenüber der Waagerechten entgegen dem Uhrzeigersinn verschwenkt. Wird das Oberschenkelteil 30 um die zweite Schwenkachse 23 entgegen dem Uhrzeigersinn verschwenkt, verringert sich der eingeschlossene Winkel zwischen der Rückseite des Oberschenkelteils 30 und der Rückseite des Unterschenkelteils 20, auch Kniewinkel genannt. Eine solche Verschwenkbewegung ist eine Knieflexion.

Die zweite Lagerstelle 42 führt eine Bewegung auf einer Kreisbahn um die zweite Schwenkachse 23 aus, wodurch neben einer Horizontalkomponente eine Vertikalkomponente in der Bewegung der zweiten Lagerstelle 42 auftritt. Aufgrund der starren Kopplung und des gleichbleibenden Abstandes zwischen der ersten Lagerstelle 41 und der zweiten Lagerstelle 42 erfolgt eine Verschwenkbewegung des Fußteils entgegen dem Uhrzeigersinn um die erste Schwenkachse 12. Ein Anheben der Fußoberseite oder des Fußrückens in Richtung auf das Unterschenkelteil 30, also das Bewegen der Fußspitze um die Schwenkachse 12 in Richtung auf das Unterschenkelteil 20, nennt sich Dorsalflexion, eine umgekehrte Bewegung, bei der die Fußsohle oder die Fußspitze in Richtung auf den Fußboden bewegt wird, nennt sich Plantarflexion. Bei einer Plantarflexion vergrößert sich der Winkel zwischen der Oberseite des Fußteils 10 und der Vorderseite des Unterschenkelteils 20, bei einer Dorsalflexion verringert sich der Winkel.

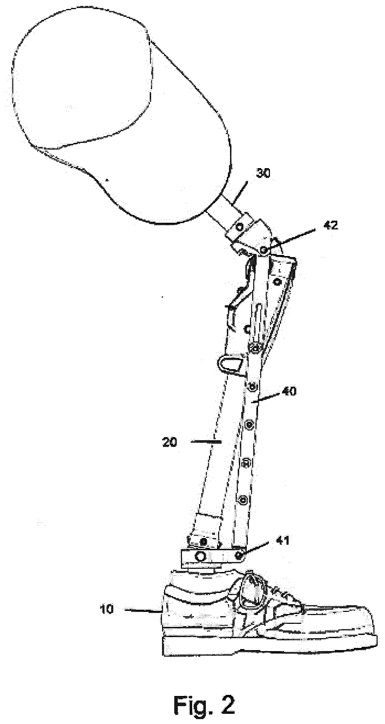

In der Figur 2 ist die Ausgestaltung der Prothese gemäß Figur 1 in einer flektierten Stellung gezeigt, in der das Oberschenkelteil 30 den Knieflexionsgrenzwinkel eingenommen hat. Zwischen dem Unterschenkelteil 20 und dem Oberschenkelteil 30 ist ein Prothesenkniegelenk angeordnet, das beispielsweise auch einen Energiespeicher und/oder eine Dämpfereinrichtung aufweisen kann. In der Stellung der Figur 2 fluchtet die Kraftübertragungseinrichtung 40 mit der Verbindungslinie zwischen der oberen Lagerstelle 42 und der Schwenkachse 23 und die maximale Vorwärtsrotation des Unterschenkelteils 20 ist erreicht.

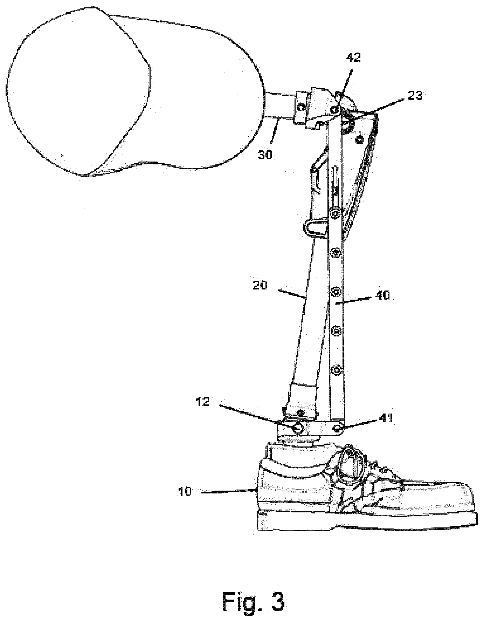

In der Figur 3 ist die orthopädietechnische Vorrichtung gemäß Figur 2 in einer weiter flektierten Stellung gezeigt, das heißt, dass das Oberschenkelteil 30 weiter in Richtung auf das Unterschenkelteil 20 um die zweite Schwenkachse 23 entgegen dem Uhrzeigersinn verschwenkt wurde. Die zweite Lagerstelle 42 ist auf der Kreisbahn um die zweite Schwenkachse 23 weiter entgegen dem Uhrzeigersinn bewegt worden. Aufgrund der zum Fußteil 10 gerichteten Bewegungskomponente der Kreisbahn wird eine Druckkraft auf das Koppelelement 40 ausgeübt, sodass bei einer Knieflexion über den Knieflexionsgrenzwinkel, der in der Stellung gemäß Figur 2 gezeigt ist, hinaus eine Kraftrichtungsumkehr und damit auch eine Bewegungsumkehr der Verschwenkbewegung des Unterteils 20 relativ zu dem Fußteil 10 um die erste Schwenkachse 12 erfolgt. Bei einer zunehmenden Knieflexion nach Erreichen des Knieflexionsgrenzwinkels wird das Unterschenkelteil 20 entgegen dem Uhrzeigersinn um die erste Schwenkachse 12 verschwenkt. Das Fußteil 10 bleibt auf dem Fußboden und die Kniegelenkachse 23 beschreibt eine Bahnkurve um die erste Schwenkachse 12 und wird aufgrund des vergleichsweise großen Abstandes der Schwenkachsen 12, 23 zueinander im Wesentlichen in Horizontalrichtung nach hinten, also entgegen der Gehrichtung verlagert. Die zweite Schwenkachse 23 wandert nach Erreichen einer maximal nach vorne bewegten Stellung bei dem Knieflexionsgrenzwinkels wieder nach hinten. Diese Bewegung ist aufgrund der vergleichsweise geringen distalen Bewegungsstrecke nach Erreichen des Scheitelpunktes bei dem Knieflexionsgrenzwinkel vergleichsweise gering, sodass in der dargestellten Position, die im Wesentlichen einer sitzenden Position entspricht, die Kniegelenksachse 23 bei einem flach aufgesetzten Fußteil 10 gegenüber einer gestreckten Stellung der orthopädietechnischen Vorrichtung leicht nach vorne verlagert ist, sodass sich das Unterschenkelteil 20 beim Sitzen leicht nach vorne neigt.

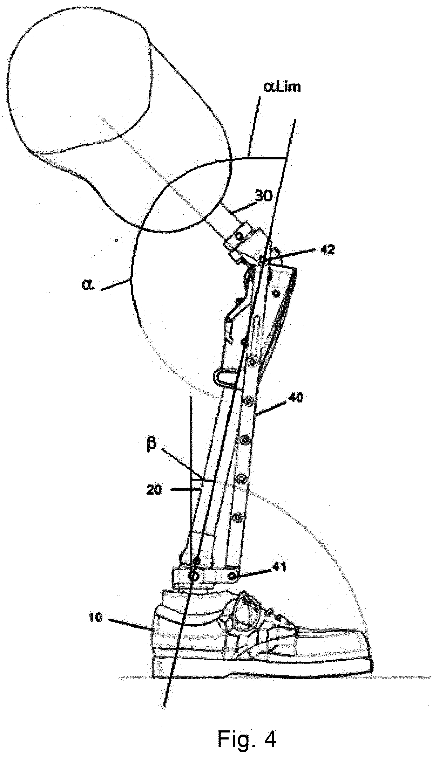

In der Figur 4 ist die Stellung gemäß Figur 2 mit den zugehörigen Winkelbeziehungen dargestellt. Der Knöchelwinkel β ist zwischen der durch die Schwenkachse 12 verlaufenden Vertikalen oder Senkrechten und der Verbindungslinie zwischen den Schwenkachsen 12, 23, die in der Figur 1 aufgeführt sind, eingezeichnet, wobei die Sichtweise von der Senkrechten durch die Schwenkachse 12 zwischen dem Fußteil 10 und dem Unterschenkelteil 20 auf dem kürzesten Weg zum Unterschenkelteil 20 eingetragen ist. Der Kniewinkel α ist zwischen der Verbindungslinie der Schwenkachsen 12, 23 und der Längserstreckung des Oberschenkelteils 30 auf der Rückseite angetragen, der Knieflexionsgrenzwinkel α_{Lim} ist der Nebenwinkel des Kniewinkels α und ergibt mit diesem zusammen 180°. In der Stellung der Figur 4 ist der Knieflexionsgrenzwinkel α_{Lim} erreicht, bei der ein maximaler Knöchelwinkel β erreicht ist. Die zweite Lagerstelle 42 befindet sich in einer maximalen Entfernung von der ersten Schwenkachse 12.

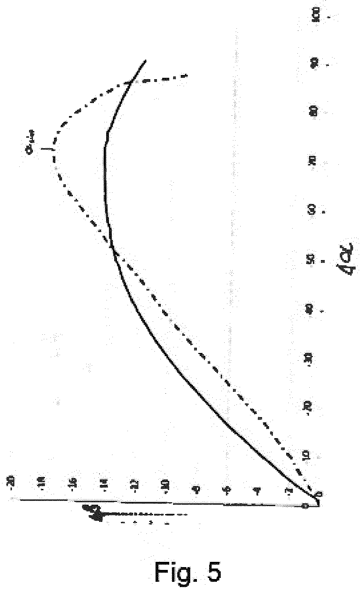

Figur 5 zeigt zwei Winkelverläufe, einen für ein gesundes Bein und einen für eine orthopädietechnische Vorrichtung gemäß der vorliegenden Erfindung, wobei der Verlauf für die orthopädietechnische Vorrichtung in einer durchgezogenen Linie und der Verlauf für ein gesundes Bein in einer gestrichelten Linie dargestellt sind. Auf der Ordinate sind die Winkelveränderungen Δβ des Knöchelgelenkwinkels ausgehend von dem minimalen Knöchelgelenkwinkel β in der maximal extendierten Stellung des Kniegelenkes aufgetragen. Die Abszisse zeigt die Veränderung des Kniewinkels α ausgehend von einer maximal extendierten Stellung, bei der der Kniewinkel α ungefähr 180° beträgt. Der Figur 5 ist zu entnehmen, dass bei einem Hinsetzen und einem Aufstehen von dem maximalen Kniewinkel α bis zu einem Kniewinkelgrenzwert der Knöchelgelenkswinkel β zunimmt. Die Kniewinkelveränderung Δα zeigt, dass bei einem abnehmenden Kniewinkel α über einen Bereich von ca. 75°, also bei einer Einbeugung um ungefähr 75°, ausgehend von der extendierten Stellung, ein Knieflexionsgrenzwinkel α_{Lim} erreicht ist. Bei diesem Knieflexionsgrenzwinkel α_{Lim} ist der Knöchelgelenkswinkel β maximal, das heißt, dass die Knöchelgelenksveränderung Δβ maximal ist. In dem dargestellten Ausführungsbeispiel in Verbindung mit der Figur 4 ist das Unterschenkelteil 20 um ca. 18° aus einer Ausgangsstellung nach vorn, also in Gehrichtung, verschwenkt. Bei einer weiteren Flexion des Kniegelenkes bis in eine angenähert waagerechte Stellung des Oberschenkelteils 30, bei der der Kniewinkel α um ca. 90° abgenommen hat, findet eine Rückverschwenkung des Unterschenkelteils 20 beim Hinsetzen oder beim Aufstehen statt, also eine Verlagerung des Unterschenkelteils 20 um die erste Schwenkachse 12 in rückwärtige Richtung, bis auf einen Verschwenkwinkel von 5° bis 10° für den Knöchelgelenkswinkel β, ausgehend von der Ausgangsposition. Dies heißt, dass bei der üblichen Sitzposition das natürliche Knöchelgelenk eine Dorsalflexion von 5° bis 10° ausführt, wenn der Fuß beim Hinsetzen nicht versetzt wird. Demgegenüber ist in der durchgezogenen Linie der Verlauf der Veränderung des Knöchelgelenkwinkels β über die Veränderung des Kniegelenkwinkels α gezeigt. Der Verlauf entspricht qualitativ dem des gesunden Beines, wie er in der gestrichelten Version gezeigt ist. Der etwas unterschiedliche, flachere Verlauf der Kurve für die orthopädietechnische Vorrichtung kann durch eine Verformung des Fußes oder des Fußteiles der orthopädietechnischen Vorrichtung ergänzt werden, so dass sich im Ergebnis eine Annäherung an den oder sogar Übereinstimmung mit dem natürlichen Verlauf ergibt. Auch bei der orthopädietechnischen Einrichtung wird bei einem Knieflexionsgrenzwinkel α_{Lim}, der im Wesentlichen mit dem Knieflexionsgrenzwinkel des gesunden Beines übereinstimmt, eine maximale Dorsalflexion erreicht. Im dargestellten Ausführungsbeispiel beträgt die Dorsalflexion 14°. Im Anschluss an den Knieflexionsgrenzwinkel α_{Lim} wird bei einer weiteren Einbeugung der Knöchelgelenkwinkel β wieder verringert, das Unterschenkelteil 20 also wieder um die erste Schwenkachse 12 in rückwärtiger Richtung verschwenkt, so dass eine angenähert natürliche Position des Unterschenkelteils 20 und des Kniegelenkes und damit auch des Oberschenkelteils 30 während des Hinsetzens und auch während des Aufstehens erreicht werden kann.

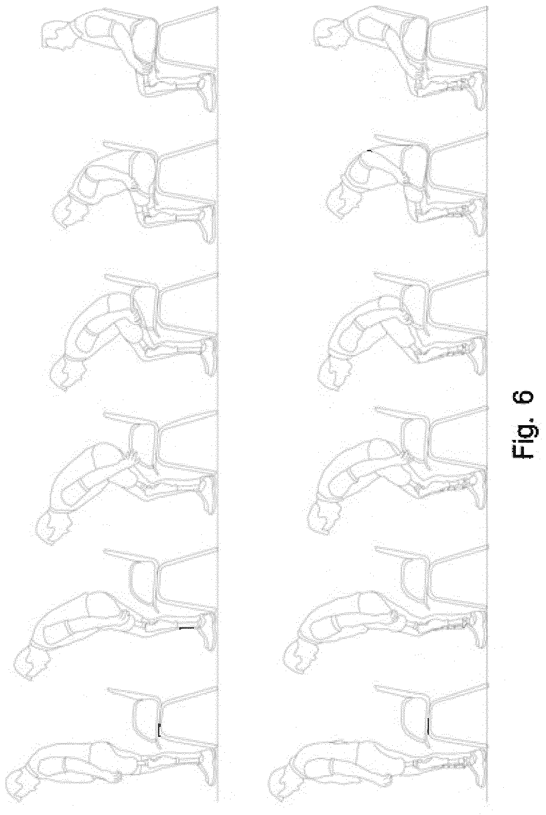

In der Figur 6 sind übereinander zwei unterschiedliche Ablaufphasen des Hinsetzens gezeigt. In den oberen Verlaufsbildern sind die sechs Phasen des Hinsetzens von der stehenden Position bis zur sitzenden Position ohne die erfindungsgemäße orthopädietechnische Vorrichtung gezeigt, in der unteren Sequenz mit der erfindungsgemäßen Vorrichtung. Von links nach rechts ausgehend von der gestreckten, maximal extendierten Stellung im Kniegelenk erfolgt eine Einbeugung und Knieflexion, bei dem das Unterschenkelteil 20 bei einem flächig aufgesetzten Fußteil im Wesentlichen senkrecht verbleibt. Der Körperschwerpunkt des Patienten wird um die Kniegelenksachse auf einer Kreisbahn bewegt und verlässt bereits in der dritten Bewegungsphase die Unterstützungsfläche durch die Füße. Dadurch kippt der ganze Körper nach hinten und der Nutzer der orthopädietechnischen Vorrichtung muss sich mit den Händen abstützen. Diese Abstützphase ist in der vierten Darstellung von links gezeigt, der Oberschenkel hat hier ungefähr einen Winkel von 70°. Das gesamte Gewicht muss von dem unversorgten Bein und den Armen aufgefangen werden. Anschließend lässt sich der Nutzer der orthopädietechnischen Vorrichtung in den Stuhl fallen und richtet sich wieder auf.

In der unteren Darstellung sind die sechs Phasen korrespondierend dargestellt. In der zweiten Darstellung von links ist zu erkennen, dass bereits bei einer leichten Knieflexion der orthopädietechnischen Vorrichtung das Unterschenkelteil nach vorne verschwenkt, so dass der Körperschwerpunkt weiter über der Unterstützungsfläche der Füße bleibt. In der dritten Bewegungsphase ist der Knöchelgelenkswinkel β weiter verringert, die Kniegelenksachse weiter nach vorne bewegt und der Körperschwerpunkt liegt im Vergleich zu einer ungekoppelten Bewegung zwischen Knieflexion und Dorsalflexion weiter frontal im Bereich der Unterstützungsfläche der Füße. Das Hinsetzen in der vierten Bewegungsphase ist wesentlich erleichtert, der Nutzer fällt nicht mit seinem Becken in die Rückenlehne, sondern setzt sich wesentlich weiter vorne auf die Sitzfläche. In der vollständig abgesenkten Position befindet sich das Unterschenkelteil 20 weiterhin in einer leicht geneigten Stellung, die einer natürlichen Position eines Unterschenkels im Wesentlichen entspricht.

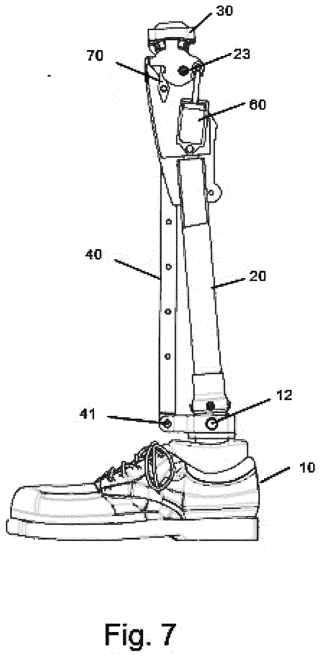

Eine Variante der Erfindung ist in der Figur 7 gezeigt, bei der eine Dämpfereinrichtung 60 in dem Unterschenkelteil 20 angeordnet ist. Durch die Lagerung einer Kolbenstange beabstandet von der zweiten Schwenkachse 23 wird eine Schwenkbewegung um die Schwenkachse 23 in eine Linearbewegung eines Zylinders in einen Kolben umgewandelt. Innerhalb der Dämpfereinrichtung 60 kann auch eine Energiespeichereinrichtung angeordnet sein, sodass sich eine Kombination aus Dämpfereinrichtung und Energiespeicher ergibt.

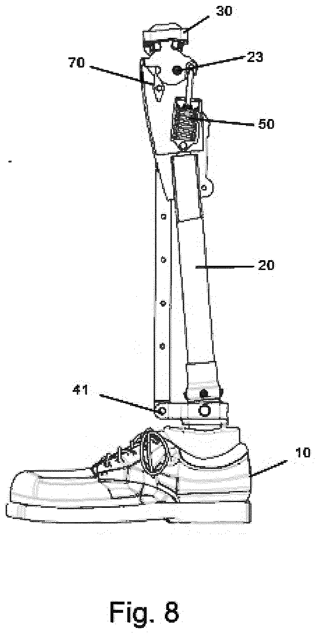

In der Figur 8 ist eine Schnittdarstellung einer Variante gemäß Figur 7 gezeigt, in der zu erkennen ist, dass das Unterschenkelteil 20 modular aufgebaut ist und aus einem Unterschenkelrohr und einem Gelenkunterteil besteht, in dem der Energiespeicher 50 angeordnet ist. Der Energiespeicher 50 in Gestalt einer Feder wird über eine Knieflexion aufgeladen, bei der das Oberteil oder Oberschenkelteil 30 um die zweite Schwenkachse 23 in Flexionsrichtung. Auf der Rückseite der zweiten Schwenkachse 23, also posterior zu der Schwenkachse 23, ist ein Bolzen gelagert, über den eine Schwenkbewegung um die Schwenkachse 23 in eine Linearbewegung zur Kompression des Energiespeichers 50 umgewandelt werden kann. Der Energiespeicher 50 kann mit einer Verriegelungseinrichtung ausgestattet sein, um wahlweise die gespeicherte Energie wieder an den Bolzen abzugeben, um eine Extensionsbewegung zu ermöglichen oder zu unterstützen.

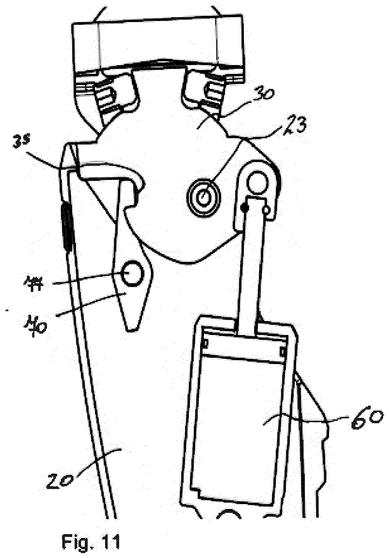

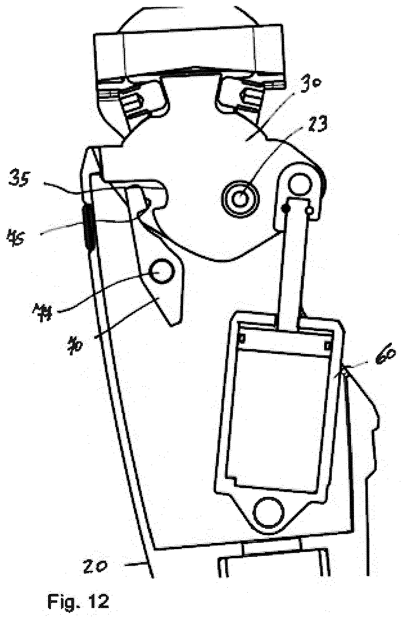

In dem dargestellten Ausführungsbeispiel der Figuren 7 und 8 ist dem Kniegelenk eine Verriegelungseinrichtung 70 in Gestalt einer Sperrklinke zugeordnet, die das Kniegelenk in der gestreckten Stellung gegen eine Flexion um die Schwenkachse 23 sperrt. Die Verriegelungseinrichtung 70 ist manuell oder motorisch betätigbar ausgebildet und wird in den Figuren 11 und 12 näher erläutert. Die Sperrklinke 70 ist in dem Unterteil oder Unterschenkelteil 20 gelagert und greift in eine Ausnehmung in dem Oberteil oder Oberschenkelteil 30 ein, um das Kniegelenk zu sperren. Um eine Flexion um die Schwenkachse 23 zu ermöglichen, wird die Sperrklinke 70 verschwenkt und außer Eingriff mit dem Oberteil oder dem Oberschenkelteil 30 gebracht.

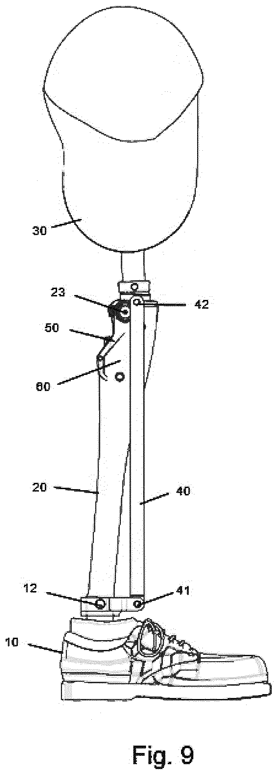

In der Figur 9 ist eine Variante der Erfindung in einer Stellung entsprechend der Figur 1 gezeigt. Im Unterschied zu der Ausgestaltung gemäß Figur 1 ist in dem Ausführungsbeispiel der Figur 9 die zweite Lagerstelle 42 nicht an einem separaten Ausleger 33 ausgebildet, sondern an dem Oberschenkelteil 30 oder einem Oberteil des Kniegelenkes. Dadurch ergibt sich ein integriertes Produkt, das bereits fertig montiert ist. Die erste Lagerstelle 12 kann an einem separaten Ausleger 11 oder an dem Fußteil 10 selbst ausgebildet sein.

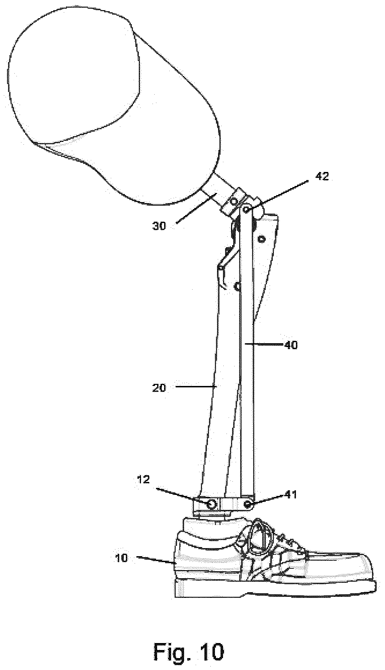

Figur 10 zeigt eine Stellung der orthopädietechnischen Vorrichtung, bei der das Oberschenkelteil 30 eine Flexionsbewegung um die zweite Schwenkachse 23 bis zu dem Knieflexionsgrenzwinkel ausgeführt hat. Das Oberschenkelteil 30 ist entgegen dem Uhrzeigersinn verschwenkt worden, die zweite Lagerstelle 42 befindet sich in einer maximalen Proximalstellung, also auf einem Scheitelpunkt der Bahnkurve. Das Fußteil 10 ist weiterhin vollflächig auf dem Boden aufgesetzt. Aufgrund der starren Kopplung zwischen der ersten Lagerstelle 41 und der zweiten Lagerstelle 42 und der Zugkraftübertragung wurde das Unterschenkelteil 20 um die Schwenkachse 12 im Uhrzeigersinn verschwenkt. Die zweite Schwenkachse 23 wurde dadurch in anteriore Richtung, also in die normale Gehrichtung nach vorne verlagert, sodass sich die zweite Schwenkachse 23 weiter vor der ersten Schwenkachse 12 befindet, in der dargestellten Stellung ungefähr auf Höhe der ersten Lagerstelle 41. Der Kniewinkel hat in der dargestellten Position einen Knieflexionsgrenzwinkel erreicht, ab dem bei einer weiteren Flexion, also bei einer weiteren Verschwenkung des Oberschenkelteils 30 entgegen der Uhrzeigerrichtung um die Schwenkachse 23, eine Bewegungsumkehr des Unterschenkelteils 20 um die erste Schwenkachse 12 ausgeführt wird. Aufgrund der in Distalrichtung wirkenden Bewegungskomponente der Kreisbahnbewegung der zweiten Lagerstelle 42 wird bei einer weiteren Flexion des Oberschenkelteils 30 eine Druckkraft von der zweiten Lagerstelle 42 über das mechanische Koppelelement 40 auf die erste Lagerstelle 41 ausgeübt. Dadurch verschwenkt das Unterschenkelteil 20 bei einer weiteren Verschwenkung entgegen dem Uhrzeigersinn um die erste Schwenkachse 12. Statt einer Befestigung des Koppelelementes 40 über einen Ausleger 33, der an dem Oberteil nachträglich befestigbar ist, ist in der Varianter der Figur 10 das Koppelelement 40 an einer Lagerstelle 42 angeordnet, die in dem Oberschenkelteil 30 oder Oberteil des Prothesenkniegelenkes integriert ist. Die Prothese ist für die Befestigung des Koppelelementes 40 ausgelegt, die notwendigen Befestigungsstellen sind bei der Konstruktion und Fertigung der Komponenten berücksichtigt.

Die Figur 11 zeigt eine Detailansicht der Figur 7 mit dem Prothesenkniegelenk, das ein Oberteil oder Oberschenkelteil 35 und das Unterteil 20 aufweist, die um eine Schwenkachse 23 verschwenkbar aneinander gelagert sind. Das Prothesenkniegelenk befindet sich in einer gestreckten Stellung. An dem Unterteil 20 ist eine Sperreinrichtung 70 in Gestalt einer Sperrklinke schwenkbar um eine Schwenkachse an einem Schwenkbolzen 77 gelagert. Die Sperrklinke 70 greift mit dem proximalen Ende in eine Ausnehmung 35 in dem Oberteil oder Oberschenkelteil 30 ein und sperrt eine Flexion um die Schwenkachse 23. Das Prothesenkniegelenk wird in der dargestellten Stellung über die Sperreinrichtung 70 gehalten, so dass keine Flexion auftreten kann. Ein ungewolltes Einbeugen des Prothesenkniegelenkes kann nicht stattfinden.

In der Figur 12 ist das Prothesenkniegelenk gemäß Figur 11 in einer entriegelten, freigegebenen, gestreckten Stellung gezeigt. Die Sperrklinke oder die Sperreinrichtung 70 ist um den Bolzen 77 entgegen dem Uhrzeigersinn verlagert worden, beispielsweise über eine mechanische Betätigung per Seilzug oder per Hebel, so dass das proximale Ende der Sperreinrichtung 70 mit dem Vorsprung 75 außer Eingriff mit der Ausnehmung 35 gebracht wird. Alternativ zu einer rein manuellen Betätigung der Sperreinrichtung 70 kann diese motorisch erfolgen, beispielsweise über einen schaltbaren Magneten, ein Elektromotor oder ähnliches. Die Betätigung kann sensorgesteuert erfolgen oder durch Betätigung eines Schalters, der an der Prothese oder über eine Fernbedienung an einem Bedienelement an einer anderen Stelle angeordnet sein kann. In der Position gemäß Figur 12 ist das Prothesenkniegelenk entriegelt und kann in Flexionsrichtung und in Extensionsrichtung verlagert werden.

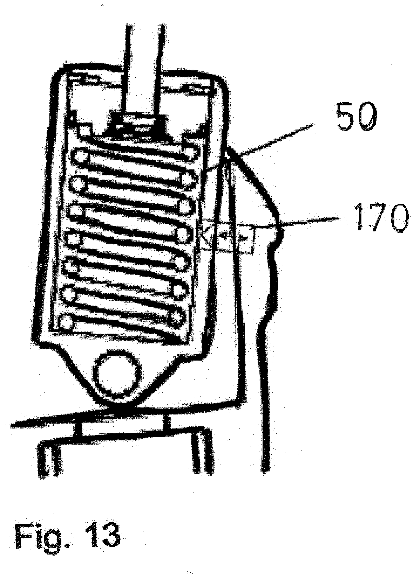

Figur 13 zeigt eine Variante der Erfindung gemäß Figur 8, bei der statt einer reinen Dämpfereinrichtung 60 ein Energiespeicher 50 zwischen dem Oberteil und dem Unterteil angeordnet ist. Der Energiespeicher 50 ist in Gestalt einer komprimierbaren Feder ausgebildet. Dem Energiespeicher 50 ist eine Verriegelungseinrichtung 170 zugeordnet, die wie in Pfeilrichtung angedeutet, in formschlüssigen Eingriff mit der Energiespeichereinrichtung 50 treten kann. Wird beispielsweise die Feder als Energiespeichereinrichtung 50 komprimiert und die Verriegelungseinrichtung 170 in den Freiraum eingefahren und blockiert eine Entspannung der Feder 50, wird eine Rückübertragung der Energie verhindert oder behindert, da sich die Feder 50 nicht oder nur teilweise entspannen kann. Erst nach Lösen der Verriegelungseinrichtung 170 wird die Feder 50 freigegeben und kann sich entspannen, um die gespeicherte Energie abzugeben und eine Extension zu unterstützen. Die Sperreinrichtung 70 für das Kniegelenk kann mit der Verrieglungseinrichtung 170 für den Energiespeicher 50 gekoppelt sein und separat betätigbar an der Prothese oder der Orthese eingerichtet sein.

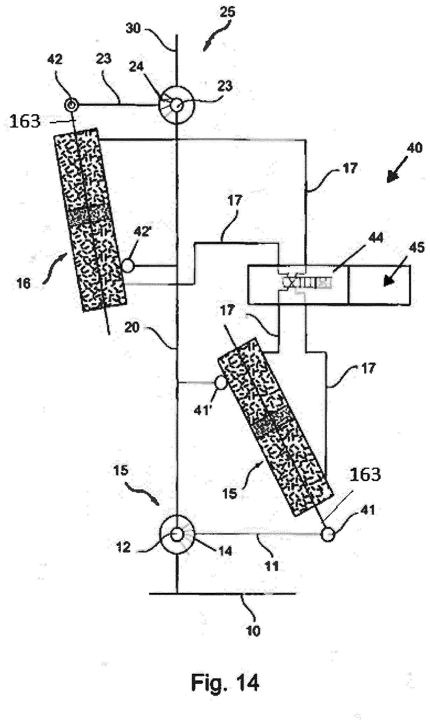

Figur 14 zeigt eine schematische Darstellung einer orthopädietechnischen Vorrichtung mit dem Fußteil 10, dem Unterschenkelteil 20 und dem Oberschenkelteil 30, die über die jeweiligen Gelenke, nämlich über das Knöchelgelenk 15 und das Kniegelenk 25, miteinander schwenkbar verbunden sind. An dem ersten Ausleger 11 ist statt einer mechanischen Kraftübertragungseinrichtung eine erste Kolben-Zylinder-Einheit 16 angeordnet, die über eine Kolbenstange 163 mit dem Ausleger 11 an der ersten Lagerstelle 41 verbunden ist. Das Gehäuse der Kolben-Zylinder-Einheit 16 ist an einer unterschenkelseitigen Lagerstelle 41'schwenkbar gelagert. Die Kolben-Zylinder-Einheit 16 unterteilt mit dem Kolben zwei Kammern. Die beiden Kammern sind über Leitungen 17 mit einem Ventilblock 44 in strömungstechnischer Verbindung gebracht. Der Ventilblock 44 weist in dem dargestellten Ausführungsbeispiel ein Dreiwegeventil auf, das über einen Aktuator 45, der mit einer Steuerung versehen ist, gekoppelt ist. Über den Aktuator 45 ist es möglich, das Dreiwegeventil in unterschiedliche Positionen zu bringen, um die Leitungen 17 unterschiedlich miteinander zu koppeln. Der Ventilblock 44 weist Ausgangsleitungen 17 auf, die zu einer zweiten Kolben-Zylinder-Einheit 16 führen, die korrespondierend zu der ersten Kolben-Zylinder-Einheit 16 aufgebaut ist. Die zweite Kolben-Zylinder-Einheit 16 ist mit ihrem Gehäuse an einer unterschenkelseitigen Lagerstelle 42'schwenkbar gekoppelt. Eine Kolbenstange 163 ist an einer zweiten Lagerstelle 42, die an einem zweiten Ausleger 33 angeordnet ist, schwenkbar gelagert. Auch die zweite Kolben-Zylinder-Einheit 16 ist durch einen Kolben in zwei Kammern unterteilt.

Sowohl an dem Kniegelenk 25 als auch an dem Knöchelgelenk 15 sind Winkelsensoren 24, 14 angeordnet oder ausgebildet, die über nicht dargestellte Leitungen oder kabellos mit der Steuerung 45 gekoppelt sind. Grundsätzlich ist es auch möglich, nur über einen Kniewinkelsensor 24 die gewünschte Funktion zu erreichen und das Verfahren durchzuführen. In Abhängigkeit von der Winkelstellung des Oberschenkelteils 30 relativ zu dem Unterschenkelteil 20 kann auf Basis von in der Steuerung 45 abgelegten Grenzwerten oder Schwellwerten eine Umschaltung des Ventilblocks 44 bewirkt werden. In der dargestellten Stellung des Ventilblocks 44 wird bei einer Knieflexion, wenn sich das Oberschenkelteil 30 entgegen dem Uhrzeigersinn um die zweite Schwenkachse 23 verschwenkt, der Kolben der mit dem zweiten Ausleger 33 verbundenen Kolben-Zylinder-Einheit 16 nach unten gedrückt und die entsprechende Zylinderkammer hinsichtlich ihres Volumens verringert. Dadurch wird Hydraulikfluid durch die Leitung 17 zu dem Ventilblock 44 geleitet. Die dargestellte Ventilstellung sieht vor, dass Hydraulikfluid aus der unteren oder distalen Kammer der oberen Kolben-Zylinder-Einheit 16 zu der unteren oder distalen Kammer der an dem ersten Ausleger 11 gekoppelten Kolben-Zylinder-Einheit geleitet wird. Dadurch wird der Kolben der unteren Kolben-Zylinder-Einheit 16 nach oben oder proximal verlagert, was zu einer Dorsalflexion des Fußteils 10 führt. Wird ein Knieflexionsgrenzwinkel erreicht und überschritten, wird das Dreiwegeventil in den Ventilblock 44 verschoben, so dass eine parallele Kopplung der Leitungen 17 in dem Ventilblock 44 erfolgt. Dadurch wird Fluid aus der unteren, distalen Kammer der oberen Kolben-Zylinder-Einheit 16 in die obere, proximale Kammer der unteren, distalen Koben-Zylinder-Einheit 16 geleitet, was dazu führt, dass die Kolbenstange 163 aus der distalen Kolben-Zylinder-Einheit 16 herausgedrückt wird. Dieses Herausdrücken bewirkt eine Verschwenkung sowohl des ersten Auslegers 11 als auch des gesamten Fußteils 10 um die erste Schwenkachse 12 und damit eine Plantarflexion.

Um eine Flexion des Oberschenkelteils 30 relativ zu dem Unterschenkelteil 20 von der Bewegung eines Fußteils 10 relativ zu dem Unterschenkelteil 20 zu entkoppeln, kann der Ventilblock 44 in eine dritte Stellung verlagert werden, bei der die beiden Kolben-Zylinder-Einheiten 16 strömungstechnisch voneinander getrennt sind. Die beiden jeweils durch einen Kolben getrennten Kammern sind dann durch eine Kurzschlussleitung miteinander gekoppelt. Sofern verstellbare Ventile in den Kurzschlussleitungen vorhanden sind, kann dann eine unabhängige Verstellung der Dämpfung in Abhängigkeit von Sensorwerten, z.B. der Winkelsensoren oder aber auch anderer Sensoren wie Kraft-, Momenten-, Raumlage-, Beschleunigungs-, Druck- und/oder Temperatursensoren

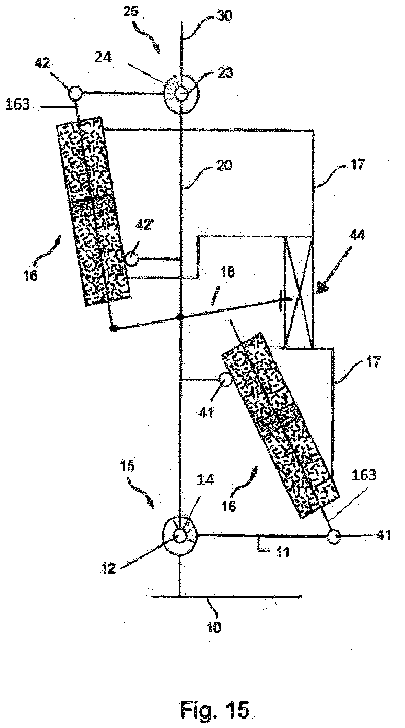

In der Figur 15 ist eine Variante der orthopädietechnischen Vorrichtung mit einer hydraulischen Kraftübertragungseinrichtung dargestellt, bei der statt einer sensorgesteuerten Aktuierung des Ventilblocks 44 eine mechanische Aktuierung vorgesehen ist. Über einen Hebel 18, der an einer aus dem Gehäuse der proximalen Kolben-Zylinder-Einheit 16 herausgeführten Kolbenstange 163 gekoppelt ist, wird über ein Umlenkelement eine mechanische Umschaltung in dem Ventilblock 44 vorgenommen, was nur schematisch dargestellt ist. Über einen Schieber oder über eine Dreheinrichtung wird dann eine entsprechende Umschaltung der Zuordnungen der distalen und proximalen Kammern der jeweiligen Kolben-Zylinder-Einheiten 16 vorgenommen, um bei Erreichen oder Überschreiten eines Knieflexionswinkels eine Umschaltung von einer Dorsalflexion des Fußteils 10 zu einer Plantarflexion zu erreichen.

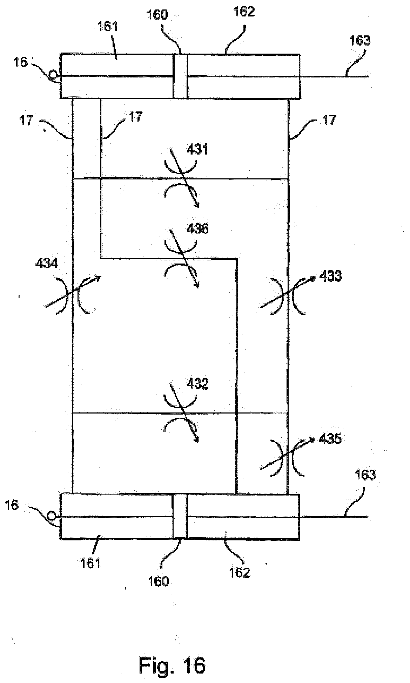

Figur 16 zeigt eine schematische Darstellung der hydraulischen Verschaltung mit mehreren Ventilen 431, 432, 433, 434, 435, 436, die einzeln auf Grundlage von Sensordaten der Sensoren 14, 24, insbesondere in Abhängigkeit von dem Kniewinkelsensor 24 aber auch in Abhängigkeit von einem der oben genannten anderen Sensortypen angesteuert werden können. Beide Kolben-Zylinder-Einheiten 16 weisen aus dem Gehäuse herausragende Kolbenstangen 163 auf, an denen ein Kolben 160 befestigt ist. Der Kolben 160 teilt den Zylinder in zwei Kammern 161, 162. Ein herausragendes Ende der Kolbenstange 163 ist an einem Ausleger 11, 33 befestigt. Das andere Ende kann entweder frei aus dem Gehäuse herausragen, wodurch Ausgleichsvolumina für die Hydraulikflüssigkeit vermieden werden können, oder an dem Kolben 160 enden. Die Hydraulikleitungen 17 verbinden die jeweils distalen und proximalen Zylinderkammern 161, 162 der beiden Kolben-Zylinder-Einheiten 16. Eine Diagonalleitung 17 verbindet eine distale Zylinderkammer 162 mit einer proximalen Zylinderkammer 161. In jeder der Hydraulikleitungen 17 ist zumindest ein Ventil 431, 432, 433, 434, 435, 436 angeordnet, um unterschiedliche Verschaltungen realisieren zu können. Werden beispielsweise die Ventile 433, 434, 435 geöffnet und die übrigen Ventile 431, 432, 436 geschlossen, ergibt sich eine Parallelschaltung, die bewirkt, dass eine Verlagerung des oberen Kolbens 160 nach links zu einer Verlagerung des unteren Kolbens 160 nach rechts führt. Um eine gegenläufige Bewegung zu erzeugen, müssen die beiden Zylinder-Kolben-Einheiten 16 kreuzweise miteinander verschaltet werden, wozu die Ventile 431, 434, 435 geschlossen werden, während die Ventile 432, 433, 436 offen stehen.

Werden die Ventile 433, 434, 436 geschlossen, führt dies zu einer Entkopplung beispielsweise der proximalen Kolben-Zylinder-Einheit 16 von der distalen Kolben-Zylinder-Einheit 16. Durch teilweises Verschließen der geöffneten Ventile 431, 432, 435 ist es möglich, den Widerstand gegen eine Verlagerung anzupassen.

Wird nur das obere Ventil 431 geöffnet, bleibt beispielsweise das Knöchelgelenk 15 starr, wohingegen das Kniegelenk 25 eingebeugt werden kann. Der Widerstand gegen die Einbeugung ergibt sich durch den hydraulischen Widerstand des geöffneten Ventils 431. Ein Steifkniegelenk 25 und ein bewegliches Knöchelgelenk 15 sind möglich, wenn die Ventile 432, 435 geöffnet und die übrigen Ventile geschlossen bleiben.

## Patentansprüche

1. Orthopädietechnische Vorrichtung mit einem Fußteil (10), einem Unterschenkelteil (20) und einem Oberschenkelteil (30), das Fußteil (10) ist mit dem Unterschenkelteil (20) über ein Knöchelgelenk (15) schwenkbar um eine erste Schwenkachse (12) verbunden, das Unterschenkelteil (20) ist mit dem Oberschenkelteil (30) über ein Kniegelenk (25) schwenkbar um eine zweite Schwenkachse (23) verbunden, das Fußteil (10) ist mit dem Oberschenkelteil (30 ) über eine Kraftübertragungseinrichtung (40) verbunden, wobei die Kraftübertragungseinrichtung (40) bei einer Knieflexion über einen ersten Knieflexionswinkelbereich eine Dorsalflexion des Fußteils (10) bewirkt, **dadurch gekennzeichnet, dass** nach Überschreiten eines Knieflexionsgrenzwinkels (α_{Lim}) bei weiterer Knieflexion die Kraftübertragungseinrichtung eine Plantarflexion des Fußteils (10) bewirkt, wobei der Knieflexionsgrenzwinkel (α_{Lim}) zwischen 50° und 80° liegt.

2. Orthopädietechnische Vorrichtung nach Anspruch 1, **dadurch gekennzeichnet, dass** die Kraftübertragungseinrichtung (40) als hydraulisches System oder als Zugkraft und Druckkraft übertragende mechanische Koppeleinrichtung ausgebildet ist.

3. Orthopädietechnische Vorrichtung nach Anspruch 2, **dadurch gekennzeichnet, dass** die mechanische Koppeleinrichtung an einer ersten Lagerstelle (41) beabstandet zu der ersten Schwenkachse (12) an dem Fußteil (10) und an einer zweiten Lagerstelle (42) beabstandet zu der zweiten Schwenkachse (23) an dem Oberschenkelteil (30) gelagert ist und die erste und zweite Lagerstelle (41, 42) bei Erreichen des Knieflexionsgrenzwinkels (α_{Lim}) eine maximal distale oder proximale Position einnimmt.

4. Orthopädietechnische Vorrichtung nach Anspruch 3, **dadurch gekennzeichnet, dass** die Position zumindest einer der Lagerstellen (41, 42) einstellbar ist.

5. Orthopädietechnische Vorrichtung nach Anspruch 3 oder 4, **dadurch gekennzeichnet, dass** die Lagerstellen (41, 42) auf einer Kreisbahn geführt sind.

6. Orthopädietechnische Vorrichtung nach einem der Ansprüche 3 bis 5, **dadurch gekennzeichnet, dass** die Lagerstellen (41, 42) abnehmbar an dem Fußteil (10) und/oder dem Oberschenkelteil (30) befestigt sind.

7. Orthopädietechnische Vorrichtung nach einem der Ansprüche 2 bis 6, **dadurch gekennzeichnet, dass** die Länge der Koppeleinrichtung einstellbar ist.

8. Orthopädietechnische Vorrichtung nach einem der voranstehenden Ansprüche, **dadurch gekennzeichnet, dass** zwischen dem Fußteil (10) und dem Unterschenkel (20) und/oder dem Unterschenkelteil (20) und dem Oberschenkelteil (30) ein Energiespeicher (50) und/oder eine Dämpfereinrichtung (60) angeordnet sind.

9. Orthopädietechnische Vorrichtung nach einem der voranstehenden Ansprüche, **dadurch gekennzeichnet, dass** dem Knöchelgelenk (12) und/oder dem Kniegelenk (23) eine betätigbare Sperreinrichtung (70) zugeordnet sind.

10. Orthopädietechnische Vorrichtung nach einem der voranstehenden Ansprüche, **dadurch gekennzeichnet, dass** sie als eine Orthese oder Prothese ausgebildet ist.

## Claims

1. An orthopedic device having a foot part (10), a lower-leg part (20) and a thigh part (30), the foot part (10) being connected by an ankle joint (15) to the lower-leg part (20) so as to be pivotable about a first pivot axis (12), the lower-leg part (20) being connected by a knee joint (25) to the thigh part (30) so as to be pivotable about a second pivot axis (23), the foot part (10) being connected to the thigh part (30) by a force-transmitting mechanism (40), whereas the force-transmitting mechanism (40) causes a dorsiflexion of the foot part (10) in the event of a knee flexion over a first knee flexion angle range, **characterized in that** in the event of further knee flexion after a knee flexion limit angle (α_{Lim}) has been exceeded, the force-transmitting mechanism causes a plantar flexion of the foot part (10), whereas the knee flexion limit angle (α_{Lim}) is between 50° and 80°.

2. The orthopedic device as claimed in claim 1, **characterized in that** the force-transmitting mechanism (40) is designed as a hydraulic system or as a mechanical coupling mechanism that transmits tensile force and compressive force.

3. The orthopedic device as claimed in claim 2, **characterized in that** the mechanical coupling mechanism is mounted on the foot part (10) at a first bearing (41) spaced apart from the first pivot axis (12) and is mounted on the thigh part (30) at a second bearing (42) spaced apart from the second pivot axis (23), and the first and second bearings (41, 42) adopt a maximum distal or proximal position when the knee flexion limit angle (α_{Lim}) is reached.

4. The orthopedic device as claimed in claim 3, **characterized in that** the position of at least one of the bearings (41, 42) is adjustable.

5. The orthopedic device as claimed in claim 3 or 4, **characterized in that** the bearings (41, 42) are guided on a circular trajectory.

6. The orthopedic device as claimed in one of claims 3 through 5, **characterized in that** the bearings (41, 42) are secured detachably on the foot part (10) and/or the thigh part (30).

7. The orthopedic device as claimed in one of claims 2 through 6, **characterized in that** the length of the coupling mechanism is adjustable.

8. The orthopedic device as claimed in one of the preceding claims, **characterized in that** an energy store (50) and/or a damper mechanism (60) are arranged between the foot part (10) and the lower leg (20) and/or between the lower-leg part (20) and the thigh part (30).

9. The orthopedic device as claimed in one of the preceding claims, **characterized in that** the ankle joint (12)and/or the knee joint (23 are assigned an actuatable locking mechanism (70).

10. The orthopedic device as claimed in one of the preceding claims, **characterized in that** it is designed as an orthosis or prosthesis.

## Revendications

1. Dispositif orthopédique comprenant une partie pied (10), une partie jambe (20) et une partie cuisse (30), la partie pied (10) étant reliée à la partie jambe (20) par une articulation de cheville (15) de manière à pouvoir pivoter autour d'un premier axe de pivotement (12), la partie jambe (20) étant reliée à la partie cuisse (30) par une articulation de genou (25) de manière à pouvoir pivoter autour d'un deuxième axe de pivotement (23), la partie pied (10) étant reliée à la partie cuisse (30) par un organe de transmission de force (40), l'organe de transmission de force (40) provoquant une flexion dorsale de la partie pied (10) lors d'une flexion du genou sur une première plage d'angle de flexion du genou,
**caractérisé en ce qu'**après dépassement d'un angle limite de flexion de genou (α_{Lim}), lors d'une flexion supplémentaire du genou, l'organe de transmission de force provoque une flexion plantaire de la partie pied (10), l'angle limite de flexion de genou (α_{Lim}) étant compris entre 50° et 80°.

2. Dispositif orthopédique selon la revendication 1,
**caractérisé en ce que** l'organe de transmission de force (40) est réalisé sous la forme d'un système hydraulique ou d'un organe de couplage mécanique transmettant une force de traction et une force de compression.

3. Dispositif orthopédique selon la revendication 2,
**caractérisé en ce que** l'organe de couplage mécanique est monté sur la partie pied (10) à un premier point de montage (41) à distance du premier axe de pivotement (12) et sur la partie cuisse (30) à un deuxième point de montage (42) à distance du deuxième axe de pivotement (23), et les premier et deuxième points de montage (41, 42) adoptent une position maximale distale ou proximale lorsque l'angle limite de flexion de genou (α_{Lim}) est atteint.

4. Dispositif orthopédique selon la revendication 3,
**caractérisé en ce que** la position de l'un au moins des points de montage (41, 42) est réglable.

5. Dispositif orthopédique selon la revendication 3 ou 4,
**caractérisé en ce que** les points de montage (41, 42) sont guidés sur une trajectoire circulaire.

6. Dispositif orthopédique selon l'une des revendications 3 à 5,
**caractérisé en ce que** les points de montage (41, 42) sont fixés de manière amovible à la partie pied (10) et/ou à la partie cuisse (30).

7. Dispositif orthopédique selon l'une des revendications 2 à 6,
**caractérisé en ce que** la longueur de l'organe de couplage est réglable.

8. Dispositif orthopédique selon l'une des revendications précédentes,
**caractérisé en ce qu'**un accumulateur d'énergie (50) et/ou un organe d'amortissement (60) sont disposés entre la partie pied (10) et la partie jambe (20) et/ou entre la partie de jambe (20) et la partie cuisse (30).

9. Dispositif orthopédique selon l'une des revendications précédentes,
**caractérisé en ce qu'**un organe de blocage (70) actionnable est associé à l'articulation de cheville (12) et/ou à l'articulation de genou (23).

10. Dispositif orthopédique selon l'une des revendications précédentes,
**caractérisé en ce qu'**il est réalisé comme une orthèse ou une prothèse.
